**Europäisches Patentamt**

**European Patent Office**

**Office européen des brevets**

(19)

(11) Veröffentlichungsnummer: **0 300 444**

**A1**

# (12) EUROPÄISCHE PATENTANMELDUNG

(21) Anmeldenummer: **88111678.4**

(22) Anmeldetag: **20.07.88**

(51) Int. Cl.⁴ **C07C 141/08 , C11D 1/37**

(30) Priorität: **24.07.87 DE 3724500**

(43) Veröffentlichungstag der Anmeldung:
**25.01.89 Patentblatt 89/04**

(84) Benannte Vertragsstaaten:
**DE FR GB IT**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Ploog, Uwe, Dr.**
**Haydnweg 5**
**D-5657 Haan(DE)**
Erfinder: **Hochschon, Wolfgang**
**Ellerstrasse 46**
**D-4010 Hilden(DE)**

(54) **Alkylsulfat-Ethersulfat-Gemische und deren Verwendung.**

㊼ Alkylsulfat-Ethersulfat-Gemische bestehen im wesentlichen aus Verbindungen der Formel

(I)     $R^1-(OC_3H_6)_x-OSO_3^{(-)} \cdot \frac{1}{n} M^{n(-)}$

in der $R^1$ eine Alkylgruppe mit 10 bis 16 C-Atomen, $x = 0$ oder eine Zahl von 1 bis 10 und M ein Alkali-, Magnesium, Ammonium-, Mono-, Di- oder Trialkanolammoniumion und n dessen Valenz ist, und welches 30 bis 80 Gew.-% Alkylsulfate der Formel I, in welcher $x = 0$ ist, enthält. Sie eignen sich zur Herstellung wäßriger Tensidzubereitungen mit guter Kältestabilität und guter Verdickbarkeit durch Elektrolytzusatz, insbesondere für flüssige Wasch- und Reinigungsmittel.

EP 0 300 444 A1

## "Alkylsulfat-Ethersulfat-Gemische und deren Verwendung"

Die Erfindung betrifft neue Waschrohstoffe vom Typ der Alkylsulfat-Ethersulfat-Gemische mit besonders günstigen anwendungstechnischen Eigenschaften.

Alkylethersulfate haben wegen ihrer herausragenden anwendungstechnischen Eigenschaften eine große Bedeutung als schaumstarke und hautfreundliche Waschrohstoffe für zahlreiche Anwendungen, insbesondere aber für flüssige Wasch- und Reinigungsmittel sowohl auf kosmetischem Gebiet als auch auf dem Gebiet der manuell anzuwendenden Haushaltsreiniger und Geschirrspülmittel erlangt. Die Produkte werden fast ausschließlich aus Ethylenoxid-Addukten an natürliche oder synthetische Fettalkohole mit 10 - 16 C-Atomen hergestellt. Dem gegenüber haben Alkylethersulfate auf Basis von Propylenoxid-Anlagerungsprodukten bisher keine kommerzielle Bedeutung erlangt, da sie allgemein schlechtere anwendungstechnische Eigenschaften aufweisen, insbesondere ein geringeres Schäumvermögen und eine geringe Verdickbarkeit der verdünnten wässrigen Lösungen durch Zusatz von Elektrolyten.

Fettalkoholsulfate haben gegenüber Alkylpolyethylenglycolethersulfaten den Nachteil einer höheren Krafttemperatur, d. h. einer geringeren Kältestabilität der verdünnten, wässrigen Lösungen. Vor allem beim Versuch, verdünnte Lösungen von Alkylsulfaten durch Elektrolytzusatz zu verdicken, wird sehr leicht eine Austrübung des Alkylsulfats und die Bildung ungelöster Sedimente erzielt. Darüber hinaus besitzen die Alkylsulfate eine ungünstigere Haut- und Schleimhautverträglichkeit.

Es wurde nun gefunden, daß die ungünstigen anwendungstechnischen Eigenschaften der Alkylsulfate einerseits und der Alkylpolypropylenglycolethersulfate andererseits in Gemischen aus diesen Tensiden nicht mehr auftreten und daß sich die Anwendungseigenschaften beider Tenside sogar in synergistischer Weise in Gemischen steigern und den anwendungstechnischen Eigenschaften von Alkylpolyethylenglycolethersulfaten sehr nah kommen.

Gegenstand der Erfindung ist daher ein Alkylsulfat-Ethersulfat-Gemisch, dadurch gekennzeichnet, daß es im wesentlichen aus Verbindungen der Formel (I)

(I) $\quad R^{\cdot}\text{-}(OC_3H_6)_x\text{-}OSO_3^{(-)} \doteq M^{n(-)}$

besteht, in der $R^{\cdot}$ eine Alkylgruppe mit 10 - 16 C-Atomen, $x = 0$ oder eine Zahl von 1 - 10 und M ein Alkali-, Magnesium-, Ammonium-, Mono-, Di- oder Trialkanolammoniumion mit 2 oder 3 C-Atomen in der Alkanolgruppe und n dessen Valenz ist, und welches 30 - 80 Gew.-% Alkylsulfate der Formel (I), in welcher $x = 0$ ist, enthält.

Die Herstellung von Alkylsulfaten der Formel (I), in der $x = 0$ ist, ist literaturbekannt und wird seit langem im großtechnischen Maßstab durchgeführt. Auch die Herstellung von Alkylpolypropylenglycolethersulfaten der Formel I, in welcher $x = 1\text{-}10$ ist, wurde bereits in der Literatur beschrieben. In "The Journal Of The Americal Oil Chemists Society", Vol 43 (1966). Seite 157 - 160 wird die Herstellung solcher Ethersulfate aus den inividuellen Etheralkoholen beschrieben. In dieser Publikation wird auch beschrieben, daß bei der Anlagerung von Propylenoxid an Fettalkohole in Gegenwart basischer Katalysatoren, wie z. B. von KOH, ein Homologengemisch verschiedener Propoxylate erhalten wird, welches auch nichtumgesetzten Ausgangsalkohol enthält, und zwar um so mehr, je weniger Propylenoxid angelagert wird.

Daraus folgt, daß bei der Sulfatierung eines technischen Propylenoxidanlagerungsprodukts eine dem nicht propoxylierten Alkohol entsprechende Menge an Alkylsulfat gebildet wird. Bei der Anlagerung von 2 Mol Propylenoxid an einen linearen Fettalkohol mit 12 - 14 C-Atomen in Gegenwart von Natriumalkoholat als Katalysator bei 170 °C wurde z. B. ein Propoxylat erhalten, welches noch ca. 24 Gew.-% nicht umgesetzten Fettalkohol enthielt. Ein daraus durch Sulfatierung mit z. B. Chlorsulfonsäure oder Schwefeltrioxid hergestelltes Alkylethersulfat erhält daher zwangsläufig ca. 27 Gew.-% Alkylsulfat.

Zur Herstellung der erfindungsgemäßen Alkylsulfat-Ethersulfat-Gemische kann man verschiedene Verfahren anwenden: Entweder man vermischt Fettalkoholpolypropylenglycolethersulfate auf Basis von Anlagerungsprodukten von 1 - 10 Mol Propylenoxid an Fettalkohole mit 10 - 16 C-Atomen, die weniger als 30 Gew.-% Fettalkoholsulfat enthalten, mit der berechneten Menge an Fettalkoholsulfat. Oder man vermischt Fettalkoholpropoxylate mit einem Gehalt von weniger als 25 Gew.-% an freiem Fettalkohol mit der berechneten Menge an freiem Fettalkohol und sulfatiert das erhaltene Gemisch nach bekannten Verfahren. Schließlich kann man an einen Fettalkohol mit 10 - 16 C-Atomen auch so wenig Propylenoxid anlagern oder die Propoxylierung unter solchen Bedingungen durchführen, daß im Propoxylat eine ausreichende Menge an freiem Fettalkohol enthalten ist und die Sulfatierung eines solchen technischen Propoxylats direkt zu den erfindungsgemäßen Alkylsulfat-Ethersulfat-Gemischen führt.

Am besten scheint die Arbeitsweise geeignet zu sein, bei der man ein Anlagerungsprodukt von 2 - 6 Mol Propylenoxid an einem Fettalkohol mit 10 - 16 C-Atomen mit so viel freiem Fettalkohol vermischt, daß sich ein Gemisch mit der berechneten Menge an freiem Fettalkohol bildet, und dann dieses Gemisch mit Chlorsulfonsäure oder Schwefeltrioxid nach bekannten Verfahren sulfa tiert. Den dabei gebildeten Schwefelsäurehalbester neutralisiert man dann mit Alkalihydroxid, z. B. mit wässriger Natronlauge, Kalilauge, Aufschlämmungen von Lithium- oder Magnesiumhydroxid, wässriger Ammoniaklösung oder wässrigen Lösungen von Monoethanolamin, Diethanolamin, Triethanolamin, Monoisoproanolamin, Diisopropanolamin oder Triisopropanolamin. Besonders günstige anwendungstechnische Eigenschaften haben erfindungsgemäße Alkylsulfat-Ethersulfat-Gemische, wenn 50 - 80 Gew.-% Alkylsulfat der Formel (I), in welcher $x = 0$ ist und 20 - 50 Gew.-% Ethersulfate der Formel (I), in welcher $x = 1 - 6$ ist, enthalten sind. Optimales Kälteverhalten und sehr gute Schäum- und Verdickungseigenschaften weisen erfindungsgemäße Alkylsulfat-Ethersulfat-Gemische auf, die 60 - 80 Gew.-% Verbindungen der Formel (I), in welcher $x = 0$ ist und 20 bis 40 Gew.-% Verbindungen der Formel (I), in welcher $x = 1 - 6$ ist, enthalten.

Die erfindungsgemäßen Alkylsulfat-Ethersulfat-Gemische weisen gute Tensideigenschaften in wässriger Lösung auf, diese Lösungen weisen Anwendungseigenschaften auf, die denen der Alkylpolyethylenglycolethersulfate sehr nahe kommen.

Ein weiterer Erfindungsgegenstand sind daher wässrige Tensidzubereitungen mit einem Gehalt von 0,1 bis 75 Gew.-% an erfindungsgemäßen Alkylsulfat-Ethersulfat-Gemischen. Zwar lassen sich auch Tensidzubereitungen mit noch höheren Anteilen an Alkylsulfat-Ethersulfat-Gemischen herstellen, diese sind jedoch sehr zäh und daher nur schwer zu handhaben.

Die erfindungsgemäßen Alkylsulfat-Ethersulfat-Gemische und die diese enthaltenden wässrigen Zubereitungen eignen sich für eine Vielzahl von Anwendungen. Bevorzugt ist dabei die Verwendung zur Herstellung von Wasch- und Reinigungsmitteln, insbesondere von flüssigen, wässrigen Wasch- und Reinigungsmitteln. Sie kön nen die weit verbreiteten Alkylpolyethylenglycolethersulfate besonders bei solchen Verwendungen ersetzen, bei welchen die menschliche Haut oder Schleimhaut mit dem Tensid in Berührung kommt, also z. B. in flüssigen Haushaltsreinigern, flüssigen Waschmitteln für manuelle Anwendungen, flüssigen, manuellen Geschirrspülmitteln, Haarshampoos, flüssigen Syndetseifen, sowie in Dusch- und Badepräparaten. Die folgenden Beispiele sollen den Gegenstand der Erfindung näher erläutern ohne ihn hierauf zu beschränken.

Beispiele

Zum Vergleich herangezogene Alkylsulfate und Alkyl-polypropylenglycolsulfat

1. $C_{12-16}$-Alkylsulfat, Na-Salz

Es wurde ein Alkylsulfat durch Sulfatierung eines $C_{12-16}$-Fettalkoholschnittes (ca. 2 Gew.-% $C_{10}$, ca. 63 % $C_{12}$, ca. 22 % $C_{14}$, ca. 12 % $C_{16}$, ca. 1 Gew.-% $C_8$, mittleres Molekulargewicht 196) mit Chlorsulfonsäure und Neutralisation des Alkylschwefelsäureesters mit wäßriger Natronlauge hergestellt. Das Produkt hatte folgende Kennzahlen:
Aktivsubstanz (Alkoholextrakt mit NaCl-Korrektur nach Einheitsmethode DGF-H-III-4):     30,5 Gew.-%
Aniontensid (Zweiphasentitration nach Einheitsmethode DGF-H-III-10):     0,983 mval/g
NaCl:     0,4 Gew.-%
$Na_2SO_4$:     0,8 Gew.-%

2. $C_{12-14}$-Alkylsulfat, Mg-Salz

Es wurde ein Handelsprodukt (Texapon®MGS, Henkel KGaA) der folgenden Zusammensetzung verwendet:
Aktivsubstanz (DGF-H-III-4):     29,0 Gew.-%
Aniontensid (DGF-H-III-10):     1,01 mval/g

$MgCl_2$:    0,5 Gew.-%

$MgSO_4$:    1,0 Gew.-%

Das Produkt basiert auf einem Fettalkohol der folgenden Zusammensetzung: ca. 1 % $C_{10}$, ca. 72 % $C_{12}$, ca. 26 % $C_{14}$, ca. 1 % $C_{16}$, mittleres Molekulargewicht: 194

### 3. $C_{12-16}$-Alkylsulfat-$NH_4^{(+)}$-Salz

Es wurde ein Handelsprodukt (Texapon®ALS, Henkel KGaA) der folgenden Zusammensetzung eingesetzt:

Aktivsubstanz (DGF-H-III-4):    28 Gew.-%

Aniontensid (DGF-H-III-10):    0,952 mval/g

$NH_4Cl$:    0,5 Gew.-%

$(NH_4)_2SO_4$:    1,4 Gew.-%

Das Produkt basiert auf einem Fettalkohol der gleichen Zusammensetzung wie Beispiel 1.

### 4. $C_{12-18}$-Alkyl + 2 PO-Sulfat, Na-Salz

Stufe 1: 642 g (3,09 Mol) eines $C_{12-18}$-Fettalkohols (ca. 2 % $C_{10}$, ca. 58 % $C_{12}$, ca. 20 % $C_{14}$, ca. 9 % $C_{16}$, ca. 11 % $C_{18}$, mittleres Molekulargewicht: 207) wurden mit 3 g einer 30%igen , methanolischen Lösung von Na-methylat versetzt und in einem Rührautoklaven bei 100 °C und einem Unterdruck von 30 mbar von Methanol befreit. Nach ca. 30 Minuten wurde mit Stickstoff gespült, ein Druck von 0,5 bar eingestellt und der Fettalkohol unter Rühren bei 170 °C portionsweise mit 358 g (6,17 Mol) Propylenoxid umgesetzt, wobei der Druck maximal bis 7 bar anstieg. Zum Schluß wurde der Autoklav für 30 Minuten bei 100 °C auf 30 mbar evakuiert. Das Propoxylat wurde in Form eines gelblichen Öls mit einer Hydroxylzahl (DGF-C-V-17a) von 178,1 erhalten. Der Gehalt an freiem Fettalkohol wurde mit 24 Gew.-% ermittelt (Gaschromatographisch).

Stufe 2: 314,3 g (1,0 Mol) des Propoxylats wurden mit 116,5 g (1,0 Mol) des Propoxylats wurden mit 116,5 g (1,0 Mol) Chlorsulfonsäure in üblicher Weise sulfatiert und der Schwefelsäurehalbester mit verdünnter, wäßriger Natronlauge (45 g NaOH in 1 000 g $H_2O$) neutralisiert. Das erhaltene $C_{12-18}$-Alkyl + 2 PO-sulfat, Na-Salz hatte die folgenden Kennzahlen:

Aktivsubstanz (DGF-H-III-4):    28,4 Gew.-%

Aniontensid (DGF-H-III-10):    0,608 mval/g

NaCl:    0,35 Gew.-%

$Na_2SO_4$:    0,45 Gew.-%

Der Gehalt des Produktes an Alkylsulfat errechnet sich zu 27 Gew.-% des Aniontensids.

### Erfindungsgemäße Alkylsulfat-Ethersulfat-Gemische

### 5. $C_{12-18}$-Alkyl + 1 PO-sulfat, Na-Salz

Stufe 1: 782 g (3,76 Mol) eines $C_{12-18}$-Fettalkohols (wie in Beispiel 4) wurde unter Verwendung von 3 g einer 30%igen methanolischen Lösung von Na-methylat als Katalysator mit 218 g (3,76 Mol) Propylenoxid umgesetzt. Das Propoxylat wurde als gelbliches Öl mit einer Hydroxylzahl (DGF-C-V-17a) von 212,6 erhalten. Der Gehalt an freiem Fettalkohol wurde mit 28 Gew.-% ermittelt (Gaschromatographisch).

Stufe 2: 395,9 g (1,5 Mol) des Propoxylats wurden mit 174,8 g (1,5 Mol) Chlorsulfonsäure sulfatiert und der Schwefelsäurehalbester mit verdünnter, wäßriger Natronlauge (63 g (1,5 Mol) NaOH in 1 322 g Wasser) neutralisiert. Das erhaltene $C_{12-18}$-Alkyl-1 PO-sulfat hatte folgenden Aniontensidgehalt:

Aniontensid (DGF-H-III-10):    0,729 mval/g

Der Gehalt des Produktes an Alkylsulfat errechnet sich zu 30,1 Gew.-% des Aniontensids.

6. Fettalkohol/Fettalkoholpropoxylat-Mischsulfat-Na-Salz

Eine Mischung aus 174,2 g (0,89 Mol) eines $C_{12-16}$-Fettalkohols (wie in Beispiel 1) und 200,5 g (0,62 Mol) eines Fettalkohol-$C_{12-18}$-2 PO-Addukts (gemäß Beispiel 4. Stufe 1) wurden mit 174,8 g (1,5 Mol) Chlorsulfonsäure sulfatiert und mit wäßriger Natronlauge (64 g NaOH in 1 270 g $H_2O$) neutralisiert.

Das (46,5 Gew.-% $C_{12-16}$-Alkyl + 53,5 Gew.-% $C_{12-18}$ + 2 PO)-sulfat, Na-Salz wurde als gelbliche Lösung mit folgenden Kennzahlen erhalten:

Aktivsubstanz (DGF-H-III-4):    28,3 Gew.-%

Aniontensid (DGF-H-III-10):    0,756 mval/g

NaCl:    0,07 Gew.-%

$Na_2SO_4$:    0,8 Gew.-%


Anwendungstechnische Prüfungen

Die Produkte der Beispiele 1 bis 6 sowie durch Mischen aus diesen Produkten hergestellte Zusammensetzungen (Beispiele 7 bis 12) wurden bezüglich Schäumverhalten, Kälteverhalten (Klarflüssigkeit) und Verdickbarkeit durch Zusatz von NaCl überprüft. Das Schäumvermögen wurde nach der Schlagschaummethode (DIN 53902) bei 40 °C, mit 1 g Aktivsubstanz/l bei 0 °dH und 20 °dH (deutsche Härtegrade) bestimmt.

Die Ergebnisse sind in den Tabellen I und II zusammengestellt. Es zeigt sich, daß die erfindungsgemäßen Produkte mit einem Anteil von 30 bis 80 Gew.-% Alkylsulfat vom Aniontensid (Akylsulfat-Ethersulfat-Gemisch), d. h. Beispiele 5 bis 12, ein besseres Schäumvermögen in hartem Wasser und eine bessere Verdickbarkeit durch Elektrolyzusatz aufweisen als die Vergleichsprodukte (Beispiele 1 bis 4).

Tabelle I

| (Schäumvermögen, Lagerverhalten) | | | | | |
|---|---|---|---|---|---|
| Nr. | Produkt | Alkylsulfat Gew.-% des Aniontensids | Schaum (ml) bei 40 °C, 1 g/l | | Lagerverhalten 24 Std. bei + 8 °C |
| | | | 0 °dH | 20 °dH | |
| 1 | $C_{12-15}$-Alkylsulfat, Na-Salz | 100 | 815 | 220 | fest, nicht fließfähig |
| 2 | $C_{12-14}$-Alkylsulfat, Mg-Salz | 100 | 865 | 350 | klar, flüssig |
| 3 | $C_{12-16}$-Alkylsulfat, $NH_4^{(-)}$-Salz | 100 | 815 | 200 | klar, flüssig |
| 4 | $C_{12-18}$-Alkyl + 2 PO-sulfat, Na-Salz | 27 | 805 | 450 | klar flüssig |
| 5 | $C_{12-18}$-Alkyl + 1 PO-sulfat, Na-Salz | 30,1 | 600 | 590 | klar flüssig |
| 6 | 46,5 Gew.-% Nr. 1 + 53,5 Gew.-% Nr. 5 | 64,7 | 750 | 750 | trüb, flüssig |
| 7 | 60 Gew.-% Nr. 1 + 40 Gew.-% Nr. 4 | 70,8 | 757 | 750 | pastös |
| 8 | 50 Gew.-% Nr. 1 + 50 Gew.-% Nr. 4 | 63,5 | 750 | 735 | pumpbar |
| 9 | 40 Gew.-% Nr. 1 + 60 Gew.-% Nr. 4 | 56,2 | 777 | 640 | leichte Ausflockung |
| 10 | 50 Gew.-% Nr. 2 + 50 Gew.-% Nr. 5 | 65,1 | 815 | 800 | klar |
| 11 | 50 Gew.-% Nr. 3 + 50 Gew.-% Nr. 5 | 65,1 | 795 | 790 | klar |

Tabelle II

| (Verdickbarkeit durch NaCl-Zusatz) | | | | | | | |
|---|---|---|---|---|---|---|---|
| Produkte | | | Viskosität bei 20 °C (Pa.s) nach Zusatz von a Gew.-% NaCl | | | | |
| | | | a = 3 | 4 | 5 | 6 | 7 |
| 1 | | 10%ig in Wasser | trübe Separation | | | | |
| 2 | | 15%ig in Wasser | 1,0 | 11,2 | > 20 (trübe) | - | - |
| 3 | | 15%ig in Wasser | - | 2,50 | 6,70 | 8,80 | 7,60 |
| 4 | | 10%ig in Wasser | 1,26 | 8,27 | 6,65 | 2,90 | - |
| 12 | Nr 1 (50 Gew.-%) + Nr.5 (50 Gew.-%) | 10%ig in Wasser | 2,80 | 1,88 | 33,72 | 25,40 | - |
| 6 | (gemäß Tabelle I) | 10%ig in Wasser | 1,20 | 14,60 | 19,56 | 10,34 | 5,27 |
| 10 | " | 10%ig in Wasser | 0,24 | 6,35 | 25,85 | 35,97 | 21,80 |
| 11 | " | 10%ig in Wasser | 0,32 | 6,24 | 11,24 | 11,80 | 6.74 |

**Ansprüche**

1. Alkylsulfat-Ethersulfat-Gemisch. dadurch gekennzeichnet. daß es im wesentlichen aus Verbindungen der Formel (I)

(I)     $R'-(OC_3H_6)_x-OSO_3^{(-)} \frac{1}{n} M^{n(-)}$

besteht. in der $R'$ eine Alkylgruppe mit 10 bis 16 C-Atomen, $x$ = 0 oder eine Zahl von 1 bis 10 und M ein Alkali-. Magnesium-. Ammonium-. Mono-. Di- oder Trialkanolammoniumion mit 2 oder 3 C-Atomen in der Alkanolgruppe und n dessen Valenz ist und welches 30 bis 80 Gew.-% Alkysulfate der Formel (I). in welcher $x$ = 0 ist, enthält.

2. Alkylsulfat-Ethersulfat-Gemisch nach Anspruch 1. dadurch gekennzeichnet. daß es 50 bis 80 Gew.-% Alkylsulfate der Formel (I). in welcher $x$ = 0 ist und 20 bis 50 Gew.-% Ethersulfate der Formel (I). in welcher $x$ = 1 bis 6 ist, enthält.

3. Alkylsulfat-Ethersulfat-Gemisch nach Anspruch 1. dadurch gekennzeichnet. daß es 60 bis 80 Gew.-% Alkylsulfate der Formel (I). in welcher $x$ = 0 ist und 20 bis 40 Gew.-% Ethersulfate der Formel (I). in welcher $x$ = 1 bis 6 ist, enthält.

4. Wäßrige Tensidzubereitungen. gekennzeichnet durch einen Gehalt an 0.1 bis 75 Gew.-% an Alkylsulfat-Ethersulfat-Gemischen gemäß Anspruch 1 bis 3.

5. Verwendung von Alkylsulfat-Ethersulfat-Gemischen gemäß Anspruch 1 bis 3 oder von wäßrigen Tensidzubereitungen gemäß Anspruch 4 zur Herstellung von Wasch- und Reinigungsmitteln, insbesondere zur Herstellung von flüssigen. wäßrigen Wasch- und Reinigungsmitteln.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|-----------|-----|-----|-----|
| A | GB-B-1 431 271 (UNILEVER) --- | | C 07 C 141/08 C 11 D 1/37 |
| A | FR-A-2 186 530 (HENKEL & CIE.) --- | | |
| A | GB-A-2 078 246 (THE PROCTOR & GAMBLE CO.) ----- | | |

RECHERCHIERTE SACHGEBIETE (Int. Cl.4)

C 07 C 141/00
C 11 D 1/00

Der vorliegende Recherchenbericht wurde für alle Patentansprüche erstellt

| Recherchenort | Abschlußdatum der Recherche | Prüfer |
|-----|-----|-----|
| DEN HAAG | 12-10-1988 | VAN GEYT J.J.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

                    

& : Mitglied der gleichen Patentfamilie, übereinstimmendes Dokument

EPO FORM 1503 03.82 (P0403)